# EUROPEAN PATENT SPECIFICATION

(11) **EP 2 579 715 B1**
(45) Date of publication and mention of the grant of the patent: **25.07.2018**
(21) Application number: 11793068.5
(22) Date of filing: 08.06.2011
(51) Int. Cl.: A61K 31/737, A61K 45/06, A61P 1/00, A61P 9/00, A61P 11/00, A61P 13/00, A61P 15/00, A61P 17/00, A61P 19/00, A61P 25/00, A61P 27/02, A61P 29/00, A61P 31/00, A61P 37/00

(54) **HYALURONANS FOR TREATING OR PREVENTING INFLAMMATION CAUSED BY A PERIODONTAL DISEASE**
HYALURONANE ZUR BEHANDLUNG ODER VORBEUGUNG VON ENTZÜNDUNGEN BEDINGT DURCH PARODONTALE ERKRANKUNGEN
HYALURONANES POUR TRAITER OU PRÉVENIR UNE INFLAMMATION PROVOQUÉE PAR UNE PATHOLOGIE PARODONTALE

(30) Priority: 23.03.2011 US 201113069860; 08.06.2010 US 352550 P
(43) Date of publication of application: 17.04.2013
(73) Proprietor: University of Utah Research Foundation, Salt Lake City, UT 84108 (US)
(72) Inventor: PRESTWICH, Glenn, D., Eastsound WA 98245 (US); OOTTAMASATHEIN, Siam, Salt Lake City UT 84106 (US); KENNEDY, Thomas, Charlote NC 28226 (US)
(74) Representative: Dehns
(86) International application number: PCT/US2011/039550
(87) International publication number: WO 2011/156445

(56) References cited:
- WO-A1-2010/130466
- WO-A1-2010/130468
- WO-A2-2005/046562
- WO-A2-2009/124266
- US-A- 4 240 163
- US-A- 4 814 437
- US-A- 5 591 724
- US-A- 5 728 391
- US-A- 5 830 913
- US-A- 5 843 919
- US-A1- 2007 054 878
- US-A1- 2009 036 523
- US-B1- 6 339 074
- US-B2- 6 828 308
- US-B2- 6 833 363
- DATABASE EMBASE [Online] ELSEVIER SCIENCE PUBLISHERS, AMSTERDAM, NL; 2005, OGAWA D ET AL: "Sulfated hyaluronic acid, a potential selectin inhibitor, ameliorates experimentally induced crescentic glomerulonephritis", XP002699794, Database accession no. EMB-2005048319 & NEPHRON - EXPERIMENTAL NEPHROLOGY 2005 CH, vol. 99, no. 1, 2005, pages e26-e32, ISSN: 1660-2129
- MITSUHIRO MATSUDA ET AL: "Therapeutic effect of sulphated hyaluronic acid, a potential selectin-blocking agent, on experimental progressive mesangial proliferative glomerulonephritis", THE JOURNAL OF PATHOLOGY, vol. 198, no. 3, November 2002 (2002-11), pages 407-414, XP055068817, ISSN: 0022-3417, DOI: 10.1002/path.1209
- 'SBIR Award ID: 93781. Sulfated Polysaccharide Derivatives for Treatment of Macular Degeneration' GLYCOMIRA, [Online] 2009, XP003031736 Retrieved from the Internet: <URL:http://www.sbir.gov/sbirsearch/detail/ 192862>
- 'SBIR Award ID: 93482. Sulfated Polysaccharide Derivatives for the Treatment of Rosacea' GLYCOMIRA, [Online] 2009, XP003131737 Retrieved from the Internet: <URL:http://www.sbir.gov/sbirsearch/detail/ 192860>
- ATSUSHI SUZUKI ET AL: "Preparation and inhibitory activity on hyaluronidase of fully O-sulfated hyaluro-oligosaccharides", GLYCOBIOLOGY, OXFORD UNIVERSITY PRESS, US, vol. 11, no. 1, 1 January 2001 (2001-01-01), pages 57-64, XP002648213, ISSN: 0959-6658

## Description

### BACKGROUND

Systemic inflammatory diseases such as multiple sclerosis, rheumatoid arthritis, osteoarthritis, diabetic nephropathy, many pulmonary disorders, periodontal disease, and chronic inflammatory skin disorders such as psoriasis, dermatitis, acne, rosacea, photo-dermal ageing are linked to RAGE-mediated signaling and plague people worldwide. To put these diseases into perspective, the National Psoriasis Foundation reports that psoriasis alone afflicts 2-3% of the world's population or approximately 125 million people. These inflammatory conditions can be aesthetically unpleasing and can create serious health issues if left untreated.

Conventionally accepted treatments of these conditions may involve UV phototherapy, corticosteroids and glucocorticoids, acitretin, cyclosporine, and methotrexate. However, each of these treatments may cause serious side effects ranging from immune suppression and liver disease to thinning skin and causing birth defects. Due to partial or complete ineffectiveness, these treatments often leave patients unsatisfied with their results.

In addition to the treatments mentioned above, heparin treatment has also been experimentally explored. Heparin, a sulfated polysaccharide, has traditionally been used almost exclusively as an anti-coagulant, but its anti-inflammatory properties are well known. Heparin and its derivatives have shown some promise in treating these inflammatory diseases. Particularly heparin and its derivatives disrupt at least three important events in inflammatory cascades. First, heparin attaches to and blocks the leukocyte integrins P- and L-selectin. Second, heparin and its derivatives reduce the inflammatory cascade by binding to and inhibiting the cationic PMN protease human leukocyte elastase and cathepsin G, which reduces proteolytic tissue injury by PMNs that escape the first heparin barrier of selectin inhibition. Third, heparin and its derivatives potentially inhibit the interaction of the receptor for advanced glycation end-products (RAGE) with its ligands.

Although heparin and its derivatives have shown promise in treating these inflammatory diseases, treatment with heparin and its derivatives exhibit several major drawbacks. First, heparin and its derivatives are porcine-derived; thus leading to concerns of cross-species transfer of viruses. Second, because of heparin's anticoagulant properties, diabetics, cancer patients, and other patients with poor coagulation status treated with this compound are at risk of excessive bleeding. Third, heparin may induce thrombocytopenia in certain individuals who produce an antibody to the complex of heparin with the cationic protein platelet factor-4 (PF-4), resulting in catastrophic platelet aggregation and generalized paradoxical arterial and venous clotting. Thus, an important unmet need is to formulate compounds which may be used to treat inflammatory diseases while avoiding the myriad of side effects seen in other treatments.

WO 2009/124266 discloses the synthesis of alkylated and fluoroalkylated semi-synthetic glycosaminoglycosan ethers and their use in the treatment of a number of inflammatory diseases and skin disorders.

### SUMMARY

Described herein is a fully sulfated hyaluronan or the pharmaceutically acceptable salt or ester thereof for use in reducing or preventing inflammation produced by a periodontal disease or gingivitis, wherein the fully saturated hyaluronan has an average molecular weight of less than 10 kDa, a degree of sulfation of 3.5 to 4.0 per disaccharide unit and 100% of the primary C-6 hydroxyl protons of the N-acetyl-glucosamine residue of hyaluronan are substituted with a sulfate group. The advantages of the invention, as defined in the appended claims, will be set forth in part in the description which follows, and in part will be obvious from the description, or may be learned by practice of the aspects described below. The advantages described below will be realized and attained by means of the elements and combinations particularly pointed out in the appended claims.

### BRIEF DESCRIPTION OF THE DRAWINGS

The accompanying drawings, which are incorporated in and constitute a part of this specification, illustrate several aspects described below.
Figure 1 shows an exemplary synthetic procedure for making partially sulfated hyaluronan by (1) partial depolymerization by controlled hydrolytic chain cleavage, (2) conversion to a tributylammonium salt, and (3) sulfation to produce the partially sulfated hyaluronan.
Figure 2 shows the protective effect of fully-sulfated hyaluronan in the mouse model of LL-37 induced cystitis. Mouse bladders pre-instilled with either FOSHA (Kewpie) or FOSHA (NVZ) show significantly reduced MPO levels. The dashed line corresponds to the MPO level in control bladders treated with saline and without inflammation (100%). Values are the mean percent levels of MPO compared to the saline control. **p*<0.05 (n=3 or 4). Error bars indicate standard error of mean.

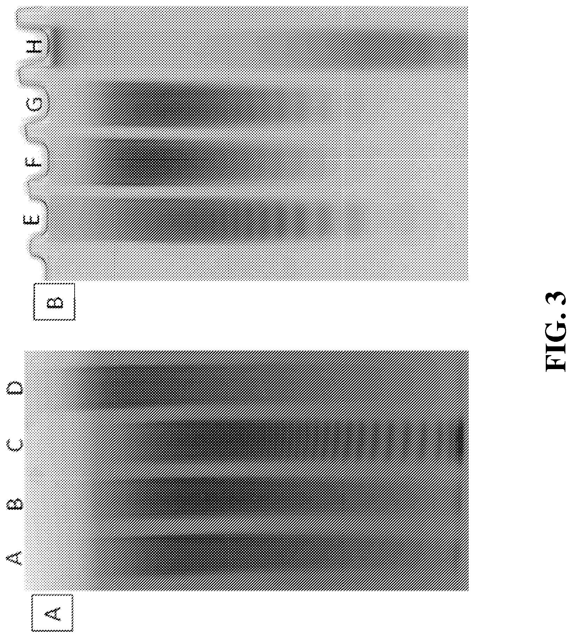

Figure 3A shows native polyacrylamide gel electrophoresis (PAGE) analysis of (A) FOS HA (2) 10 kDa, (B) FOS HA (1)10 kDa, (C) FOS BHA, and (D) POS BHA. A 10 µg aliquot of each sample was separated on a Novex® Tris-Glycine 18% gel (Invitrogen, Carlsbad, CA) run at 125 V for 1.5 hours under native conditions. Figure 3B shows native PAGE analysis of (E) FOS HA manufactured from Kewpie's Hyalo Oligo HA using pyridine-sulfur trioxide complex, (F) FOS HA manufactured from Novozymes HA using pyridine-sulfur trioxide complex, (G) POS HA manufactured from Novozymes HA using pyridine-sulfur trioxide complex, and (H) FOS HA 10 kDa manufactured from sulfur trioxide N,N-dimethylformamide complex . An aliquot (15 µg) of each sample was separated on a 20% acrylamide IDSmart Gel (Boca Scientific, Boca Raton, FL) run at 125 V for 75 minutes under native conditions. Gels were stained in an aqueous solution of 0.08% azure A.
Figure 4 shows the sulfation and C-pyridinylation of hyaluronan to produce a by-product associated with the production of partially or fully sulfated hyaluronan using the sulfur trioxide-pyridine complex.
Figure 5 is the ¹H NMR spectrum of FOSHA-Kewpie-Pyr.SO₃, which reveals three peaks between 8.00 ppm and 9.10 ppm representing the pyridinium protons of the pyridinium adduct.

### DETAILED DESCRIPTION

In this specification and in the claims that follow, reference will be made to a number of terms that shall be defined to have the following meanings:
It must be noted that, as used in the specification and the appended claims, the singular forms "a," "an" and "the" include plural referents unless the context clearly dictates otherwise. Thus, for example, reference to "a pharmaceutical carrier" includes mixtures of two or more such carriers, and the like.
"Optional" or "optionally" means that the subsequently described event or circumstance can or cannot occur, and that the description includes instances where the event or circumstance occurs and instances where it does not. For example, the phrase "optionally substituted lower alkyl" means that the lower alkyl group can or can not be substituted and that the description includes both unsubstituted lower alkyl and lower alkyl where there is substitution.

References in the specification and concluding claims to parts by weight, of a particular element or component in a composition or article, denotes the weight relationship between the element or component and any other elements or components in the composition or article for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

A weight percent of a component, unless specifically stated to the contrary, is based on the total weight of the formulation or composition in which the component is included.

A residue of a chemical species, as used in the specification and concluding claims, refers to the moiety that is the resulting product of the chemical species in a particular reaction scheme or subsequent formulation or chemical product, regardless of whether the moiety is actually obtained from the chemical species. For example, hyaluronan that contains at least one -OH group can be represented by the formula Y-OH, where Y is the remainder (i.e., residue) of the hyaluronan molecule.

The term "treat" as used herein is defined as maintaining or reducing the symptoms of a pre-existing condition. The term "prevent" as used herein is defined as eliminating or reducing the likelihood of the occurrence of one or more symptoms of a disease or disorder. The term "inhibit" as used herein is the ability of the compounds described herein to completely eliminate the activity or reduce the activity when compared to the same activity in the absence of the compound.

Described herein is a fully sulfated hyaluronan or the pharmaceutically acceptable salt or ester thereof for use in reducing or preventing inflammation produced by a periodontal disease or gingivitis according to claim 1. Reference is also made herein to partially sulfated hyaluronan or the pharmaceutically acceptable salt or ester thereof. The term "partially sulfated hyaluronan" as used herein is when the hyaluronan has a degree of sulfation less than 3.5 per disaccharide unit. The use of partially sulfated hyaluronan does not form part of the invention. The term "fully sulfated hyaluronan" as used herein is when the hyaluronan has a degree of sulfation of 3.5 to 4.0 per disaccharide unit. The terms "low molecular hyaluronan" and "hyaluronan oligosaccharide" are defined below. Examples of suitable counterions are provided below.

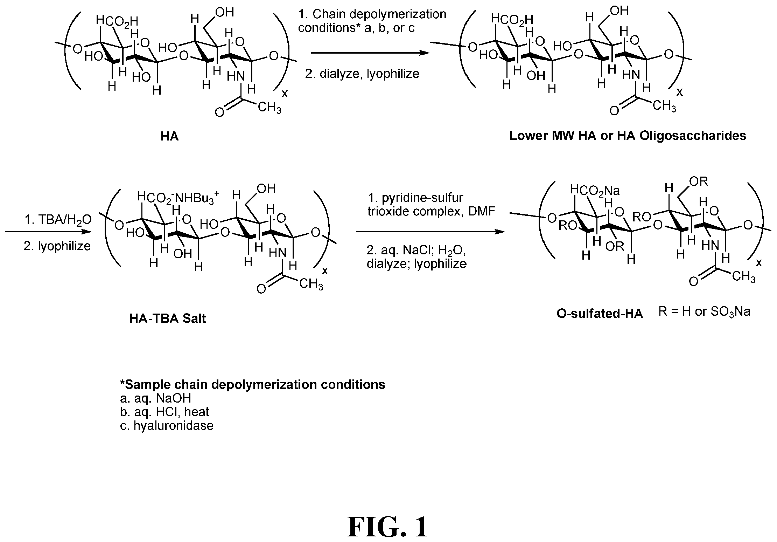

The hyaluronan starting material can exist as the free acid or the salt thereof. Derivatives of hyaluronan starting material can also be used herein. The derivatives include any modification of the hyaluronan prior to sulfation. A wide variety of molecular weight hyaluronans can be used herein for the depolymerization step. In one aspect, the hyaluronan has a molecular weight greater than 1,000 kDa prior to depolymerization. In another aspect, the hyaluronan can have a molecular weight of 10 kDa to 1,000 kDa prior to depolymerization. A wide variety of hyaluronan molecular weights can also be employed for the sulfation step. In one aspect, the hyaluronan starting material can be converted to low molecular hyaluronan or a hyaluronan oligosaccharide prior to sulfation to produce the partially or fully sulfated hyaluronan. As will be discussed in greater detail below, low molecular weight hyaluronan is hyaluronan that has been degraded with an acid or base. Alternatively, hyaluronan oligosaccharide is produced by degrading hyaluronan with an enzyme such as, for example, hyaluronan synthase or hyaluronidase in a controlled fashion. Subsequently, hyaluronan oligosaccharides having different molecular weights can be separated by GPC or ion exchange separation. Figure 1 depicts exemplary procedures for producing low molecular hyaluronan or hyaluronan oligosaccharide from hyaluronan.

In one aspect, the low molecular hyaluronan or hyaluronan oligosaccharide being sulfated has a molecular weight from 1 kDa to 2,000 kDa. In another aspect, the low molecular hyaluronan or hyaluronan oligosaccharide being sulfated has a molecular weight from 5 kDa to 10 kDa. Exemplary procedures for preparing low molecular weight hyaluronan are provided in the Examples. As discussed above, the molecular weight of the hyaluronan can be modified by cleaving hyaluronan with an acid or base to produce lower molecular weight hyaluronan. In certain aspects, the hyaluronan starting material or a derivative thereof is not derived from an animal source. In these aspects, the hyaluronan can be derived from other sources such as bacteria. For example, a recombinant *B. subtilis* expression system can be used to produce the hyaluronan starting material.

After the low molecular hyaluronan or hyaluronan oligosaccharide has been treated with a base, it is reacted with a sulfating agent to produce the partially or fully sulfated hyaluronan. Sulfating agents commonly used in organic synthesis can be used herein. Examples of sulfating agents include, but are not limited to, pyridine-sulfur trioxide complex or the triethylamine-sulfur trioxide complex. An exemplary synthetic procedure for making partially sulfated hyaluronan is provided in Figure 1. Referring to Figure 1, low molecular hyaluronan or hyaluronan oligosaccharide is converted to the tributylamine salt, lyophilized, resuspended in dimethylformamide, and subsequently treated with a sulfating agent (*e.g*., pyridine-sulfur trioxide complex) to sulfate one or more hydroxyl protons. The fully sulfated hyaluronan can be produced by adding a sufficient amount of base and sulfating agent to sulfate all of the hydroxyl groups.

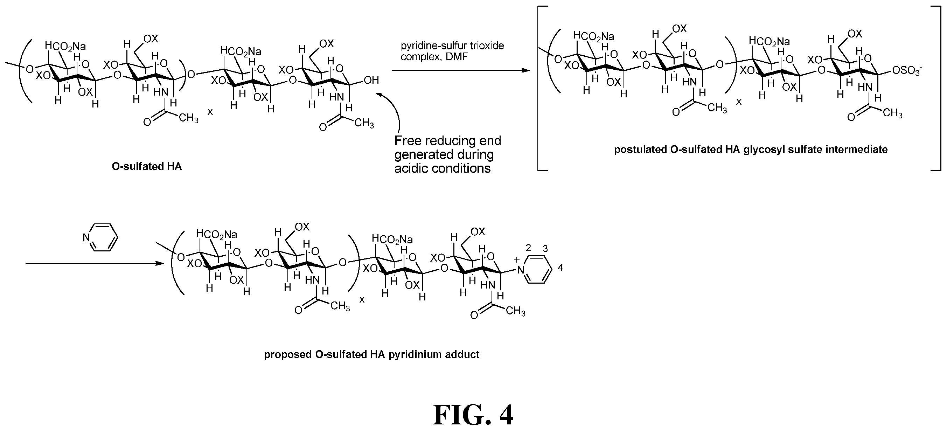

In one aspect, when the sulfating agent is a pyridine-sulfur trioxide complex, a pyridinium adduct of the partially or fully sulfated hyaluronan is produced. This is depicted in Figure 4, where pyridine is covalently attached to the partially or fully sulfated hyaluronan. Partially sulfated hyaluronan does not form part of the invention. Not wishing to be bound by theory, when hyaluronan is reacted with the pyridine-sulfur trioxide complex in a solvent such as, for example, DMF, a small amount of acid is produced from traces of water present *in situ*, which causes partial depolymerization resulting in a free reducing end group (*e.g.,* a hydroxyl group in Figure 4). The hydroxyl group of the hemiketal can ultimately be sulfated to produce a sulfated intermediate, which subsequently reacts with free pyridine produced *in situ* to produce the pyridinium adduct. In one aspect, the molecular weight of the pyridinium adduct of the fully sulfated hyaluronan is less than or equal to 10kDa. In other aspects, the molecular weight is 0.1 kDa, 0.5 kDa, 1 kDa, 2 kDa, 3 kDa, 4 kDa, 5 kDa, 6 kDa, 7 kDa, 8 kDa, 9 kDa, or 10 kDa, where any value can for the lower and upper end-point of a molecular weight range.

The average molecular weight of the fully sulfated hyaluronan is less than 10 kDa, or less than 5 kDa. In another aspect, the fully sulfated hyaluronan has an average molecular size from 0.5 kDa to less than 10 kDa, 2 Da to 10 kDa, or 3 kDa to 10 kDa. In a further aspect, the fully sulfated hyaluronan has an average molecular size from 0.5 kDa to 10 kDa or 1 kDa to 5 kDa. Depending upon reaction conditions, one or more different hydroxyl groups present in the low molecular hyaluronan or hyaluronan oligosaccharide can be sulfated. The primary C-6 hydroxyl proton of the N-acetyl-glucosamine residue of the low molecular hyaluronan or hyaluronan oligosaccharide is sulfated. In another aspect, the primary C-6 hydroxyl proton of the N-acetyl-glucosamine residue of hyaluronan and at least one C-2 hydroxyl proton or C-3 hydroxyl proton of a uronic acid residue or at least one C-4 hydroxyl proton of an N-acetyl-glucosamine residue is substituted with a sulfate group. In another aspect, the primary C-6 hydroxyl proton of the N-acetyl-glucosamine residue of the low molecular hyaluronan or hyaluronan oligosaccharide and at least one C-2 hydroxyl proton and C-3 hydroxyl proton of a uronic acid residue and at least one C-4 hydroxyl proton of an N-acetyl-glucosamine residue is substituted with a sulfate group. In another aspect, 0.001%, 0.01%, 0.1%, 1%, 5%, 10%, 20%, 30%, 40%, 50%, 60%, 70%, 80%, 90%, 95%, or less than 100%, or any range thereof of hydroxyl protons present on the low molecular hyaluronan or hyaluronan oligosaccharide can be deprotonated and subsequently sulfated.

The fully sulfated hyaluronan described herein can be the pharmaceutically acceptable salt or ester thereof. In some aspects, the pharmaceutically acceptable ester can be a prodrug. For example, free hydroxyl groups of the fully sulfated hyaluronan can be partially esterified with palmitoyl chloride to afford an amphiphilic compound that is hydrolyzed by endogenous esterases to liberate the free fully sulfated hyaluronan. Other prosthetic groups that liberate non-toxic byproducts familiar to those skilled in the art may also be used. Pharmaceutically acceptable salts are prepared by treating the free acid with an appropriate amount of a pharmaceutically acceptable base. Representative pharmaceutically acceptable bases are ammonium hydroxide, sodium hydroxide, potassium hydroxide, lithium hydroxide, calcium hydroxide, magnesium hydroxide, ferrous hydroxide, zinc hydroxide, copper hydroxide, aluminum hydroxide, ferric hydroxide, isopropylamine, trimethylamine, diethylamine, triethylamine, tripropylamine, benzalkonium, choline, ethanolamine, 2-dimethylaminoethanol, 2-diethylaminoethanol, lysine, arginine, histidine, and the like. In one aspect, the reaction is conducted in water, alone or in combination with an inert, water-miscible organic solvent, at a temperature of from about 0 °C to about 100 °C such as at room temperature. The molar ratio of fully sulfated hyaluronan to base used is chosen to provide the ratio desired for any particular salt. For preparing, for example, the ammonium salts of the free acid starting material, the starting material can be treated with approximately one equivalent of pharmaceutically acceptable base to yield a neutral salt. In other aspects, choline salts of the fully sulfated hyaluronan can be prepared and used herein.

The fully sulfated hyaluronan described herein can be formulated in any excipient the biological system or entity can tolerate to produce pharmaceutical compositions. Examples of such excipients include, but are not limited to, water, aqueous hyaluronic acid, saline, Ringer's solution, dextrose solution, Hank's solution, and other aqueous physiologically balanced salt solutions. Nonaqueous vehicles, such as fixed oils, vegetable oils such as olive oil and sesame oil, triglycerides, propylene glycol, polyethylene glycol, and injectable organic esters such as ethyl oleate can also be used. Other useful formulations include suspensions containing viscosity enhancing agents, such as sodium carboxymethylcellulose, sorbitol, or dextran. Excipients can also contain minor amounts of additives, such as substances that enhance isotonicity and chemical stability. Examples of buffers include phosphate buffer, bicarbonate buffer and Tris buffer, while examples of preservatives include thimerosol, cresols, formalin and benzyl alcohol. In certain aspects, the pH can be modified depending upon the mode of administration. For example, the pH of the composition is from about 5 to about 6, which is suitable for topical applications. Additionally, the pharmaceutical compositions can include carriers, thickeners, diluents, preservatives, surface active agents and the like in addition to the compounds described herein.

The pharmaceutical compositions can also include one or more active ingredients used in combination with the fully sulfated hyaluronan. The resulting pharmaceutical composition can provide a system for sustained, continuous delivery of drugs and other biologically-active agents to tissues adjacent to or distant from the application site. The biologically-active agent is capable of providing a local or systemic biological, physiological or therapeutic effect in the biological system to which it is applied. For example, the agent can act to control and/or prevent infection or inflammation, enhance cell growth and tissue regeneration, control tumor growth, act as an analgesic, promote anti-cell attachment, reduce alveolar bone and tooth loss, inhibit degeneration of cartilage and weight bearing joints, and enhance bone growth, among other functions. Additionally, any of the compounds described herein can contain combinations of two or more pharmaceutically-acceptable compounds. Examples of such compounds include, but are not limited to, antimicrobial agents, antiinflammatory agents, anesthetics, and the like.

The pharmaceutical compositions can be prepared using techniques known in the art. In one aspect, the composition is prepared by admixing the fully sulfated hyaluronan herein with a pharmaceutically-acceptable compound and/or carrier. The term "admixing" is defined as mixing the two components together so that there is no chemical reaction or physical interaction. The term "admixing" also includes the chemical reaction or physical interaction between the compound and the pharmaceutically-acceptable compound. Covalent bonding to reactive therapeutic drugs, e.g., those having nucleophilic groups, can be undertaken on the compound. Second, non-covalent entrapment of a pharmacologically active agent in a crosslinked polysaccharide is also possible. Third, electrostatic or hydrophobic interactions can facilitate retention of a pharmaceutically-acceptable compound in the compounds described herein.

It will be appreciated that the actual preferred amounts of the fully sulfated hyaluronan in a specified case will vary according to the specific compound being utilized, the particular compositions formulated, the mode of application, and the particular situs and subject being treated. Dosages for a given host can be determined using conventional considerations, e.g. by customary comparison of the differential activities of the subject compounds and of a known agent, e.g., by means of an appropriate conventional pharmacological protocol. Physicians and formulators, skilled in the art of determining doses of pharmaceutical compounds, will have no problems determining dose according to standard recommendations (Physicians Desk Reference, Barnhart Publishing (1999).

The pharmaceutical compositions described herein can be administered in a number of ways depending on whether local or systemic treatment is desired, and on the area to be treated. Administration for periodontal disease or gingivitis can be topically via delivery of a gel, paste, or rinse to the diseased gums or periodontal pockets. The active ingredient can also be formulated as a coating on floss, periodontal brushes, periodontal probes or other oral health care devices. Formulations for topical administration can include ointments, lotions, creams, gels, drops, suppositories, sprays, liquids and powders. Conventional pharmaceutical carriers, aqueous, powder or oily bases, thickeners and the like can be necessary or desirable. Administration can also be directly into the lung by inhalation of an aerosol or dry micronized powder. Administration can also be by direct injection into the inflamed or degenerating joint space.

Preparations for administration include sterile aqueous or non-aqueous solutions, suspensions, and emulsions. Examples of non-aqueous carriers include water, alcoholic/aqueous solutions, emulsions or suspensions, including saline and buffered media. Parenteral vehicles, if needed for collateral use of the disclosed compositions and methods, include sodium chloride solution, Ringer's dextrose, dextrose and sodium chloride, lactated Ringer's, or fixed oils. Intravenous vehicles, if needed for collateral use of the disclosed compositions and methods, include fluid and nutrient replenishers, electrolyte replenishers (such as those based on Ringer's dextrose), and the like. Preservatives and other additives can also be present such as, for example, antimicrobials, anti-oxidants, chelating agents, and inert gases and the like.

Dosing is dependent on severity and responsiveness of the condition to be treated, but will normally be one or more doses per day, with course of treatment lasting from several days to several months or until one of ordinary skill in the art determines the delivery should cease. Persons of ordinary skill can easily determine optimum dosages, dosing methodologies and repetition rates.

The fully sulfated hyaluronan is used for reducing or preventing inflammation produced by a periodontal disease or gingivitis. The fully sulfated hyaluronan and pharmaceutical compositions described herein can be placed directly in or on any biological system without purification as it is composed of biocompatible materials. Examples of sites the fully sulfated hyaluronan can be placed include, but are not limited to, soft tissue such as muscle or fat; hard tissue such as bone or cartilage; areas of tissue regeneration; a void space such as periodontal pocket; surgical incision or other formed pocket or cavity. The fully sulfated hyaluronan can be biodegradable and naturally occurring enzymes will act to degrade them over time. Components of the fully sulfated hyaluronan can be "bioabsorbable" in that the components of the partially sulfated hyaluronan will be broken down and absorbed within the biological system, for example, by a cell, tissue and the like.

In the case of dental disorders such as periodontal disease, the fully sulfated hyaluronan can be added as a component of a mouthwash or formulated into creams or gingival packing materials to be applied directly to the gingival crevice.

The fully sulfated hyaluronan and compositions described herein can deliver at least one pharmaceutically-acceptable compound to a patient in need of such delivery, comprising contacting at least one tissue capable of receiving the pharmaceutically-acceptable compound with one or more compositions described herein. The fully sulfated hyaluronan can be used as a carrier for a wide variety of releasable biologically active substances having curative or therapeutic value for human or non-human animals. Many of these substances that can be carried by the fully sulfated hyaluronan are discussed above. Included among biologically active materials which are suitable for incorporation into the gels of the invention are therapeutic drugs, e.g., anti-inflammatory agents, anti-pyretic agents, steroidal and non-steroidal drugs for anti-inflammatory use, hormones, growth factors, contraceptive agents, antivirals, antibacterials, antifungals, analgesics, hypnotics, sedatives, tranquilizers, anticonvulsants, muscle relaxants, local anesthetics, antispasmodics, antiulcer drugs, peptidic agonists, sympathiomimetic agents, cardiovascular agents, antitumor agents, oligonucleotides and their analogues and so forth. A biologically active substance is added in pharmaceutically active amounts.

In one aspect, the fully sulfated hyaluronan described herein can inhibit the activity of the receptor for Advanced Glycation Endproducts (RAGE), P-selectin, L-selectin, human leukocyte elastase, or any combination thereof.

RAGE is a cell surface receptor, expressed on a range of cells, including endothelium, fibroblasts and mononuclear phagocytes. In diabetic patients, hyperglycemia leads to non-enzymatic glycation and oxidation of proteins and lipids to form advanced glycation end products (AGEs). In smokers, AGE products accumulate in the front of the mouth, the lungs, and systemically. Smokers are also highly susceptible to focal gum diseases, periodontitis, and tooth loss from inflammatory disease. AGEs are ligands for the RAGE receptor, and ligation leads to an inflammatory cascade. In murine diabetic models, it has been shown this ligation provides a central role in oral infection, exaggerated inflammatory response, and destruction of alveolar bone leading to tooth loss. Expression of RAGE in gingival tissues has been demonstrated in patients with chronic periodontitis with and without type 2 diabetes. Efforts to block periodontal RAGE may be best performed via local delivery.

The fully sulfated hyaluronan described herein are antagonists to RAGE-mediated inflammation. As RAGE ligation is a fundamentally prominent mechanism of perpetuating inflammatory signaling, inhibiting ligation and thus suppressing up-regulation of RAGE is an effective treatment to reduce this form of inflammatory cell perturbation. The compounds described herein block the RAGE receptor, keeping ligands from binding and activating the RAGE inflammatory mechanism. Low molecular weight and water soluble sulfated hyaluronan lack anti-coagulant activity, and are thus generally considered safe for systemic and topical uses. In gingivitis, periodontitis, and post-implantation of dental implants, a locally administered fully sulfated hyaluronan could lead to the preservation of gingival tissue and bone.

The ability of the fully sulfated hyaluronan to block RAGE makes it particularly valuable as a therapeutic agent for inflammation.

The fully sulfated hyaluronan and compositions described herein are safer than other related therapies. For example, heparin and other sulfated polysaccharides can reduce diabetic complications in both animal and clinical studies, and are particularly effective against diabetic nephropathy. However, heparins cannot be used in general clinical settings to prevent diabetic complications because the anticoagulant properties present an excessive risk of bleeding. The fully sulfated hyaluronan and compositions described herein possess low anticoagulant activity, which is an important consideration for long-term treatment, which is demonstrated below in the Examples. Additionally, the partially or fully sulfated hyaluronan and compositions have little to no toxicity, which is also demonstrated in the Examples.

### EXAMPLES

The following examples are put forth so as to provide those of ordinary skill in the art with a complete disclosure and description of how the compounds, compositions, and methods described and claimed herein are made and evaluated, and are intended to be purely exemplary and are not intended to limit the scope of what the inventors regard as their invention. Efforts have been made to ensure accuracy with respect to numbers (e.g., amounts, temperature, etc.) but some errors and deviations should be accounted for. Unless indicated otherwise, parts are parts by weight, temperature is in °C or is at ambient temperature, and pressure is at or near atmospheric. There are numerous variations and combinations of reaction conditions, e.g., component concentrations, desired solvents, solvent mixtures, temperatures, pressures and other reaction ranges and conditions that can be used to optimize the product purity and yield obtained from the described process. Only reasonable and routine experimentation will be required to optimize such process conditions.

In the following examples, any references to partially sulfated hyaluronan do not form part of the invention.

### I. Preparation of Sulfated Low Molecular Weight Hyaluronan (LMW-HA) from Base-Treated Hyaluronan

### a. Base-treated LMW HA

HA (2 g, 67 kDa) was dissolved in 20 mL of NaOH (40% w/v) in a 100 mL beaker and the mixture was stirred for 2 h at room temperature to partially depolymerize the HA by inducing strand cleavage. The resulting viscous liquid was transferred to a 400 mL beaker that contained 100 mL of isopropanol and stirred for 24 h at room temperature. The resulting solution was gravity filtered (filter paper) and the crude product was collected, dissolved in 250 mL of distilled water, and the pH was adjusted to 7.0. The solution was dialyzed against distilled water for 24 h, changing the water bath 4 times during this period, and then lyophilized to dryness to obtain 1.2 g of the base-treated HA. The size of this product can be determined by HPLC< GPC or electrophoresis, and is generally in the range of 5 kDa to 20 kDa.

### b. LMW Partially O-Sulfated Base-treated HA

To obtain the tributylamine (TBA) salt of LMW HA, 0.2 mL of TBA was added to base-treated HA (0.2 g) in 20 mL of distilled water. The mixture was stirred vigorously and lyophilized to dryness. The resulting salt (LMW HA-TBA) was dissolved in 20 mL of N,N-dimethylformamide (DMF) to which the required excess (6 mol/equivalent of total hydroxyl groups in HA, 4 per disaccharide) of pyridine-sulfur trioxide complex (0.325 g) was added. After 3 hours at 40 °C, the reaction was quenched by addition of 20 mL of water, and the crude material was precipitated by adding 30 mL of cold ethanol saturated with anhydrous sodium acetate. The crude sulfated product was collected by filtration, dissolved in distilled water (30 mL) and dialyzed against 100 mM NaCl solution (changing solution six times) and against water (change water two times) for two days, changing the solution four times a day, and lyophilized to dryness. The yield of the product was 61% (0.22 g). Based on ¹H NMR, the degree of substitution was approximately 0.5-1. Elemental analysis gave a sulfur content of 4.13 %. The average molecular weight was determined by GPC to be 6,100, and the polydispersity was 2.3.

### c. LMW Fully O-Sulfated Base-treated HA

To obtain the tributylamine (TBA) salt of HA, 0.2 mL of TBA was added to base-treated HA (0.2 g) in 20 mL of distilled water. The mixture was stirred vigorously and lyophilized to dryness. The resulting salt (LMW HA-TBA) was dissolved in 20 mL of N,N-dimethylformamide (DMF) to which the required excess (16 mol/equivalent of available hydroxy group in HA) of pyridine-sulfur trioxide complex (11.0 g) was added. After 3 h at 40 °C, the reaction was quenched by the addition of 20 mL of water and the crude product was precipitated by adding 30 mL of cold ethanol saturated with anhydrous sodium acetate. The sulfated product was collected by filtration, dissolved in distilled water (30 mL), and dialyzed against 100 mM NaCl solution (changing solution six times) and against water (change water two times) for two days, changing the solution four times a day, and lyophilized to dryness to give 0.26 g of product (60% yield). The product was characterized by ¹H NMR and showed an approximate substitution degree of about 3.5. Elemental analysis gave a sulfur content of 13.22%. The average molecular weight was determined by GPC to be 5,900, with a polydispersity of 2.2.

### II. Preparation of Sulfated Low Molecular Weight Hyaluronan (LMW-HA) from Acid-Treated Hyaluronan

### a. Fully O-Sulfated Low MWHA (F-OSHA(1)-10, 000)

To obtain the tributylamine (TBA) salt of HA, 0.2 mL of TBA was added to HA (0.2 g, ca. 10,000 Da, degraded from 1.3 MDa HA) in 20 mL of distilled water. The mixture was mixed vigorously and lyophilized to dryness. The resulting salt (HA-TBA) was dissolved in 20 mL of N,N-dimethylformamide (DMF) to which the required excess (6 mol/equivalent of available hydroxyl groups in HA) of pyridine-sulfur trioxide complex (0.325 g) was added. After 3 h at 40 °C, the reaction was quenched by the addition of 20 mL of water and the crude material was precipitated by adding 30 mL of cold ethanol saturated with anhydrous sodium acetate. The sulfated product was collected by filtration, dissolved in distilled water (30 mL), and dialyzed against 100 mM NaCl solution (changing solution six times) and against water (change water two times) for two days, changing the solution four times a day, and lyophilized to dryness to give 0.19 g of product (58% yield). Elemental analysis gave a sulfur content of 12.62%, indicating sulfation of 3.0-3.5. The molecular weight was less than 3,000 Da, suggesting acidic depolymerization during sulfation and workup.

### b. Fully O-Sulfated Low MW HA (F-OSHA (2)-10,000)

To obtain the tributylamine (TBA) salt of HA, 0.2 mL of TBA was added to acid-modified 10,000 MW HA (0.2 g) in 20 mL of distilled water. The mixture was mixed vigorously and lyophilized to dryness. The resulting salt (HA-TBA) was dissolved in 20 mL of DMF to which the required excess (16 mol/equivalent of available hydroxyl group in HA) of pyridine-sulfur trioxide complex (1.1 g) was added. After 3 hours at 40 °C, the reaction was quenched by addition of 20 mL of water and the crude material was precipitated by adding 30 mL of cold ethanol saturated with anhydrous sodium acetate. The sulfated product was collected by filtration, dissolved in distilled water (30 mL), and dialyzed against 100 mM NaCl solution (changing solution six times) and against water (change water two times) for two days, changing the solution four times a day, and lyophilized to give 0.23 g of product (62% yield). The product was characterized by ¹H NMR and showed a substitution degree of 3.0 - 3.5. Elemental analysis gave a sulfur content of 12.10%. The molecular weight was less than 3,000 Da.

### c. Fully O-Sulfated Low MW HA (Kewpie Hyalo-Oligo - Pyr.SO₃)

Kewpie Hyalo-Oligo HA (200 mg, 0.5 mmole, 8.4 kDa) was dissolved in 10 mL of DMF. TBA (1 eq. 0.5 mmole, 0.12 mL) was added while stirring and stirred for an additional 10 minutes. The required excess (6 mol/equivalent of available hydroxyl groups in HA) of pyridine-sulfur trioxide complex (24 eq. 12 mmole, 1.916 g) was added. After stirring for 3 hours at 40 °C, the reaction was quenched by the addition of 15 mL of water, and the crude material was precipitated by adding 25 mL of cold ethanol saturated with anhydrous sodium acetate. The sulfated product was collected by filtration, dissolved in 25 mL of distilled water and dialyzed against 100 mM NaCl solution (changing solution six times) and against water (changing water two times) for two days, changing the solution four times a day, and lyophilized to dryness to give 0.175 g of product (51% yield). The product was characterized by ¹H NMR and showed a substitution degree of greater than 3.5. The average molecular weight was determined by GPC to be 6,800 Da with a polydispersity of 1.88.

### .d. Fully O-Sulfated Low MWHA (Novozymes- Pyr.SO₃)

Novozymes HA (200 mg, 0.5 mmole) degraded to 11 kDa was dissolved in 10 mL of DMF. TBA (1 eq. 0.5 mmole, 0.12 mL) was added while stirring, and the mixture was stirred for an additional 10 minutes. The required excess (6 mol/equivalent of available hydroxyl groups in HA) of pyridine-sulfur trioxide complex (24 eq. 12 mmole, 1.916 g) was next added. After stirring for 3 hours at 40 °C, the reaction was quenched by the addition of 15 mL of water, and the crude material was precipitated by adding 25 mL of cold ethanol saturated with anhydrous sodium acetate. The sulfated product was collected by filtration, dissolved in 25 mL of distilled water and dialyzed against 100 mM NaCl solution (changing solution six times) and against water (changing water two times) for two days, changing the solution four times a day, and lyophilized to dryness to give 0.194 g of product (57% yield). The product was characterized by ¹H NMR and showed a substitution degree of about 3.0-3.5. The average molecular weight was determined by GPC to be 8,100 Da with a polydispersity of 2.00.

### e. Partially O-Sulfated Low MW HA (Novozymes -Pyr.SO₃)

Novozymes HA (400 mg, 1.0 mmole) degraded to 11 kDa was dissolved in 25 mL of DMF. TBA (1 eq. 1.0 mmole, 0.24 mL) was added while stirring, and stirred for an additional 10 minutes. The required excess (3 mol/equivalent of available hydroxyl groups in HA) of pyridine-sulfur trioxide complex (12 eq. 12 mmole, 1.908 g) was added. After stirring for 3 hours at 40 °C, the reaction was quenched by the addition of 30 mL of water, and the crude material was precipitated by adding 50 mL of cold ethanol saturated with anhydrous sodium acetate, and then collected by filtration. The resulting crude partially O-sulfated HA was dissolved in 40 mL of distilled water and dialyzed against 100 mM of NaCl solution (changing solution six times) and against water (changing water two times) for two days, changing the solution four times a day, and lyophilized to dryness to give 0.386 g of product (56% yield). The product was characterized by ¹H NMR and showed a substitution degree of 2.0. The average molecular weight was determined by GPC to be 9,500 Da with a polydispersity of 1.77.

### e. Fully O-Sulfated Low MWHA (Novozymes -DMF.SO₃)

Novozymes HA (200 mg, 0.5 mmole) degraded to 11 kDa was dissolved in 10 mL of DMF. TBA (1 eq. 0.5 mmole, 0.12 mL) was added while stirring, and the mixture was stirred for an additional 10 minutes. The required excess (6 mol/equivalent of available hydroxyl groups in HA) of DMF-sulfur trioxide complex (24 eq. 12 mmole, 1.836 g) was added. After stirring for 3 hours at 30 °C, the reaction was quenched by the addition of 15 mL of water, and the crude material was precipitated by adding 25 mL of cold ethanol saturated with anhydrous sodium acetate, and then collected by filtration. The resulting crude fully O-sulfated HA was dissolved in 25 mL of distilled water and dialyzed against 100 mM of NaCl solution (changing solution six times) and against water (changing water two times) for two days, changing the solution four times a day, and lyophilized to dryness to give 0.057 g of product (17% yield). The product was characterized by ¹H NMR and showed a substitution degree of about 3.0-3.5. The average molecular weight was determined by GPC to be 1,900 Da with a polydispersity of 2.48.

Figure 3a shows native polyacrylamide gel electrophoresis (PAGE) analysis of (A) FOS HA (2) 10 kDa, (B) FOS HA (1) 10 kDa, (C) FOS BHA, and (D) POS BHA. 10 µg of each sample was separated on a Novex® Tris-Glycine 18% gel (Invitrogen, Carlsbad, CA) run at 125 V for 1.5 hours under native conditions. Figure 3b shows native PAGE analysis of (E) FOS HA manufactured from Kewpie's Hyalo Oligo HA using pyridine-sulfur trioxide complex, (F) FOS HA manufactured from Novozymes HA using pyridine-sulfur trioxide complex, (G) POS HA manufactured from Novozymes HA using pyridine-sulfur trioxide complex, and (H) FOS HA 10 kDa manufactured from sulfur trioxide N,N-dimethylformamide complex. 15 µg of each sample was separated on a 20% acrylamide IDSmart Gel (Boca Scientific, Boca Raton, FL) run at 125 V for 75 minutes under native conditions. Gels were stained in an aqueous solution of 0.08% azure A.

### III. In Vitro Studies

### a. Human Leukocyte Elastase (HLE) Inhibition Assay

To investigate the inhibitory effects of sulfated HA on leukocyte elastase, 100 µl of 7.5 µg/ml HLE was incubated with 100 µl of sulfated HA's at a range of concentrations from 0.001 to 100 µg/mL. The mixture was incubated for 10 minutes at 25 °C, after which 50 µl of the HLE substrate suc-Ala-Ala-Val-*p*NA (1.5 mM) was added. Active HLE cleaves the substrate and produces chromogenic *p*NA which is followed by measuring the change in absorbance at 405 nm using a kinetic read. IC₅₀ values are obtained (Table 1) using a 4-parameter logistic non-linear regression equation of the Vmax (rate of absorption) versus sulfated HA concentration.

**Table 1. Inhibition of Human Leukocyte Elastase (HLE)**

| **Sample** | **IC₅₀ value (µg/ml)** |
|---|---|
| POS BHA | 0.30 |
| FOS BHA | 0.18 |
| F-OSHA(2) 10k | 0.23 |
| F-OSHA(1) 10k | 0.22 |
| FOSHA Kewpie | 0.46 |
| FOSHA Novozymes | 0.45 |
| POSHA Novozymes | 0.47 |
| FOSHA Novozymes DMF | 0.76 |

### b. CML-BSA/RAGE complex inhibition assay

The CML-BSA and RAGE complex inhibition assay was prepared by coating a polyvinyl chloride plate with 100 µl of 5 µg/ml CML-BSA. Separately, a 1 µg/ml solution of RAGE-Fc chimera in PBST-0.1% BSA was incubated with an equal volume of serially diluted sulfated low molecular weight hyaluronan and HA oligosaccharides at concentration ranges of 0.0005 µg/ml to 100 µg/ml overnight at 4° C. The following day, 50 µl of RAGE-sulfated HA mix was transferred to each respective ligand-coated well and incubated at 37 °C for 1 hour. Wells were then washed four times with PBST. To detect bound RAGE, 50 µl of 0.5 µg/ml of anti-RAGE antibody was added to each well. The plate was incubated for 1 hour at room temperature and the wells washed again four times with PBST. HRP-conjugated secondary antibody (50 µl per well) was added, wells were incubated for 1 hour at room temperature and then washed four times with PBST. A colorimetric reaction was initiated by addition of 100 µl of TMB and terminated with the addition of 50 µl of 1 N HCl. Absorbance at 450 nm was plotted against the sulfated HA concentration and IC₅₀ values obtained (Table 2) using a 4-parameter logistic non-linear regression equation.

**Table 2. Inhibition of CML-BSA binding to RAGE**

| **Sample** | **IC₅₀ value (µg/ml)** |
|---|---|
| POS BHA | >100 |
| FOS BHA | 0.0468 |
| F-OSHA(2) 10k | 0.0176 |
| F-OSHA(1) 10k | 0.0197 |
| FOSHA Kewpie | 0.037 |
| FOSHA Novozymes | 0.023 |
| POSHA Novozymes | 0.066 |
| FOSHA Novozymes DMF | 0.521 |

### c. Characterization of the Pyridinium Adduct

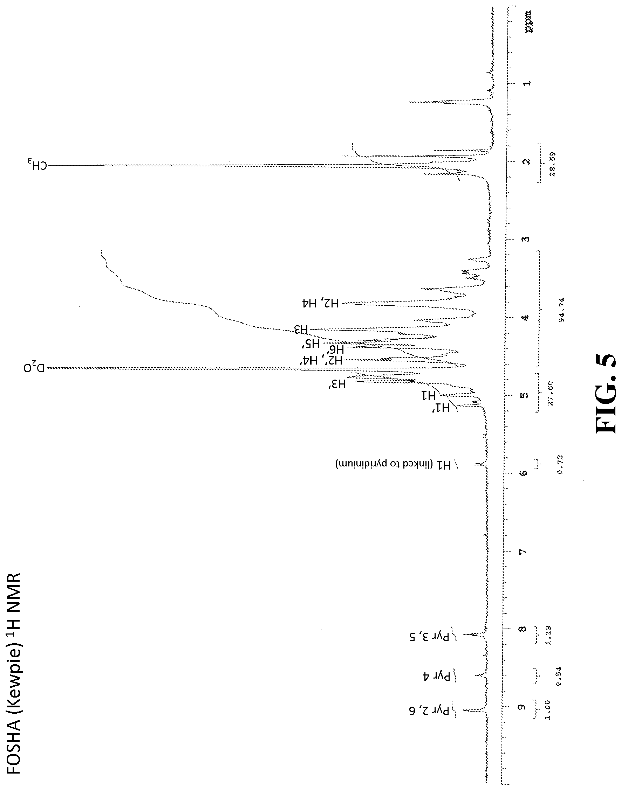

The pyridinium content of the sulfated HA samples prepared using the pyridine-sulfur trioxide complex was analyzed via UV absorbance. A standard curve was created using 1-butylpyridinium bromide (Sigma, St. Louis, MO) and the sulfated HA samples were diluted from 2 to 0.025 mg/ml as necessary for the UV measurements to fall within the standard curve. Absorbance values at 255 nm in a quartz cuvette were recorded for the standards and samples. From the standard curve, weight percent pyridinium values were calculated for each sample and are included in Table 3. The pyridinium adduct of FOSHA-Kewpie-Pyr.SO₃ was also characterized by ¹³C NMR spectroscopy and compared to published data. (Hintze V, Moeller S, Schnabelrauch M, Beirbaum S, Viola M, Worch H, Scharnweber D. "Modifications of Hyaluronan Influence the Interaction with Human Bone Morphogenetic Protein-4 (hBMP-4)" Biomacromolecules 10:3290-3297, 2009) The ¹³C NMR data is presented in Table 4. Finally, Figure 5 is the ¹H NMR spectrum of FOSHA-Kewpie-Pyr.SO₃, which reveals three peaks between 8.00 ppm and 9.10 ppm representing the pyridinium protons of the pyridinium adduct.

**Table 3. Measurement of Pyridinium Content of Sulfated HA Created with Pyridine-Sulfur Trioxide Complex**

| **Sample** | **Average wt% pyridinium** | **Standard deviation** |
|---|---|---|
| FOSHA Kewpie | 0.80 | 0.02 |
| FOSHA Novozymes | 0.462 | 0.009 |
| POSHA Novozymes | 0.662 | 0.006 |
| POS BHA | 0.11 | 0.03 |
| FOSBHA | 1.7 | 0.1 |
| F-OSHA(2) 10k | 14.7 | 0.8 |
| F-OSHA(1) 10k | 9.0 | 0.6 |

**Table 4. ¹³C NMR Data of FOSHA-Kewpie-Pyr.SO₃ compared with published data.**

| | | | | |
|---|---|---|---|---|
| 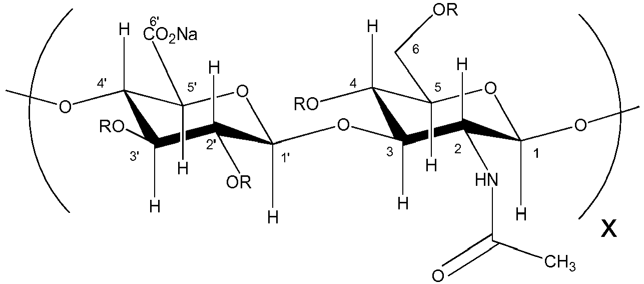 | | | | |

| | **Hya** | **sHya 1.0 (6.6% S)** | **sHya2.8 (13.1% S)** | **FOSHA-Kewpie** |
|---|---|---|---|---|
| C=O | 175.4 | 175.2 | 175.3 | 174.9 |
| C'=O (6') | 174.4 | 174.2 | 175.0 | 173.9 |
| C1' | 103.6 | 103.5 | 102.0 | 101.4 |
| C1 | 101.0 | 101.3 | 100.6 | 100.4 |
| C3 | 83.5 | 82.5 | 79.3-76.9 | 79.1-75.6 |
| C4' | 80.7 | 81.4 | | |
| C5' | 77.1 | 76.8 | | |
| C5 | 76.2 | 74.2 | | |
| C3' | 74.4 | 73.8 | | |
| C2' | 73.3 | 72.9 | 73.7 | 73.0 |
| C4 | 69.4 | 68.8 | 69.5 (small) | 68.6 |
| C6 | 61.5 | 67.5 | 68.3 | 67.7 |
| C2 | 54.9 | 54.7 | 56.0 | 55.2 |
| CH₃ | 23.2 | 22.9 | 23.6 | 23.2 |
| Py 2,6 | | | | 142.7 |
| Py 4 | | | | 128.2 |
| Py 3,5 | | | | 103.4 |

### IV. In Vivo Studies (not part of the invention)

### a. Mouse Bladder Inflammation Model

Mouse bladders are sensitive to various inflammatory substances including LL-37 (cathelicidin peptide) and have served as an excellent animal model to study the potential therapeutic agents in inflammatory diseases including cystitis. In order to investigate the anti-inflammatory effects of sulfated HA, the protective effects of FOSHA derivatives were measured in a murine cystitis model. First, C57/BL6 adult female mice were anesthetized and a catheter was inserted into the bladder through the urethra. The bladders were washed by infusing and draining 0.9 % sterile saline. Bladders were then pre-instilled with either 150 µL of saline, 10 mg/ml FOSHA (Kewpie) or 10 mg/ml FOSHA (Novozymes) for 1 hour. The bladders were emptied and then instilled with 150 µL of 320 µM LL-37 for an additional 1 hour. All animals were fully recovered without complications. Twenty-four hours after the completion of the procedure, the bladders were removed, photographed, and frozen for MPO analysis.

### b. Myeloperoxidase (MPO) assay

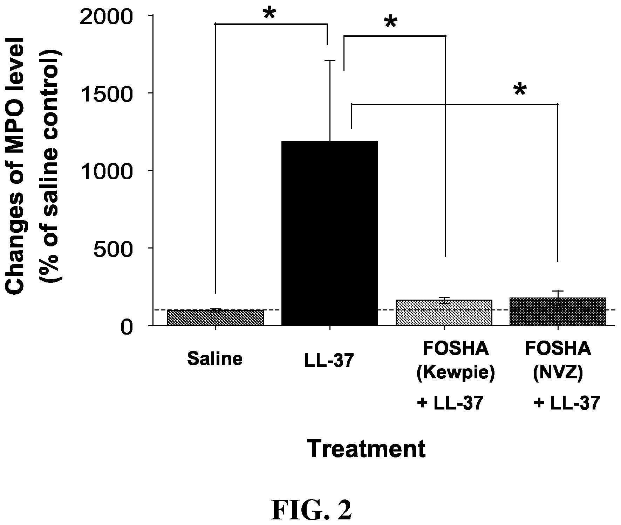

The major cellular responses to inflammatory substances are secretion of various cytokines from damaged cells that recruit various immune cells to the target site. MPO is a peroxidase enzyme expressed abundantly in polymorphonuclear cells, which are primary cells recruited to the site of inflammation during the early stage of inflammation and therefore, MPO is an excellent marker to quantitatively measure the degree of inflammation. To analyze the anti-inflammatory effects of fully-sulfated HA in the murine cystitis model, the quantity of expressed MPO was measured in the bladders pre-treated with sulfated HA and compared with the levels of expressed MPO in untreated bladders and a saline control. Bladders were weighed and homogenized. The homogenized samples were centrifuged at 5,000 rpm to separate the soluble fraction from tissue debris and the concentration of MPO in the tissue homogenates (ng/mg tissue) was measured using the mouse MPO ELISA kit (HK210, Hycult biotech, The Netherlands) and expressed as percent difference from the saline instilled control (normal bladder without inflammation). Results are provided in Figure 2.

To determine whether pretreatments of sulfated HA reduce tissue MPO concentration induced by instilled LL-37, we performed statistical analysis using one-way ANOVA followed by Tukey-Kramer multiple comparisons test using GraphPad InStat software (Version 3.1, GraphPad Software, Inc.). Statistical significance was set at *p*<*0.0.*

### c. Conclusions

The results from the *in vitro* studies demonstrate the importance of the degree of sulfation with respect to RAGE antagonist activities (Table 2). Partial sulfation (less than 6% sulfur) results in a much less potent RAGE antagonist. Sulfated HA compounds with a MW less than 2,000 Da also show reduced potency in the *in vitro* assays. Fully-sulfated HA compounds, including those possessing the pyridinium adduct in excess of 1% w/w, showed good *in vivo* efficacy in reducing inflammation in the mouse model of bladder inflammation.

## Claims

1. A fully sulfated hyaluronan or the pharmaceutically acceptable salt or ester thereof, or a combination thereof for use in reducing or preventing inflammation produced by a periodontal disease or gingivitis, wherein the fully sulfated hyaluronan has an average molecular weight of less than 10 kDa, a degree of sulfation of 3.5 to 4.0 per disaccharide unit and 100% of the primary C-6 hydroxyl protons of the N-acetyl-glucosamine residue of hyaluronan are substituted with a sulfate group.

2. The hyaluronan according to claim 1 for use according to claim 1, wherein the fully sulfated hyaluronan comprises one pyridine molecule covalently attached to the sulfated hyaluronan.

## Patentansprüche

1. Vollständig sulfatierte Hyaluronsäure oder ein pharmazeutisch akzeptables Salz oder Ester davon, oder eine Kombination davon zur Benutzung zur Verringerung oder zur Verhinderung von Entzündungen, die durch eine parodontale Erkrankung oder Gingivitis erzeugt wird, wobei die vollständig sulfatierte Hyaluronsäure ein durchschnittliches Molekulargewicht von weniger als 10 kDA aufweist, einen Sulfatierungsgrad von 3,5 bis 4,0 pro Disaccharideinheit und 100% der primären C-6 Hydroxylprotonen des N-Acetyl-Glucosaminrestes der Hyaluronsäure durch eine Sulfatgruppe ersetzt sind.

2. Hyaluronsäure nach Anspruch 1 zur Benutzung nach Anspruch 1, wobei die vollständig sulfatierte Hyaluronsäure ein Pyridinmolekül umfasst, das kovalent an die sulfatierte Hyaluronsäure gebunden ist.

## Revendications

1. Hyaluronane complètement sulfaté ou sel ou ester pharmaceutiquement acceptable de celui-ci ou combinaison de celui-ci pour utilisation dans la réduction ou la prévention d'une inflammation produite par une parodontolyse ou une gingivite, dans lequel le hyaluronane complètement sulfaté a un poids moléculaire moyen de moins de 10 kDa, un degré de sulfatation de 3,5 à 4,0 par unité de disaccharide et 100 % des protons d'hydroxyle C-6 primaires du résidu de N-acétyl-glucosamine du hyaluronane sont substitués par un groupe sulfate.

2. Hyaluronane selon la revendication 1 pour utilisation selon la revendication 1, dans lequel le hyaluronane complètement sulfaté comprend une molécule de pyridine fixée par covalence au hyaluronane sulfaté.
